(19) Europäisches Patentamt
European Patent Office
Office européen des brevets

(11) Numéro de publication: **0 389 359 A1**

# DEMANDE DE BREVET EUROPEEN

(21) Numéro de dépôt: 90400762.2

(22) Date de dépôt: 20.03.90

(51) Int. Cl.⁵: C07D 257/02, C07F 9/6524, C07F 11/00

(30) Priorité: 20.03.89 FR 8903600

(43) Date de publication de la demande: 26.09.90 Bulletin 90/39

(84) Etats contractants désignés: AT BE CH DE DK ES FR GB GR IT LI LU NL SE

(71) Demandeur: CENTRE NATIONAL DE LA RECHERCHE SCIENTIFIQUE (CNRS)
15, Quai Anatole France
F-75007 Paris(FR)

(72) Inventeur: Handel, Henri
30, rue E. Corbière
F-29200 Brest(FR)
Inventeur: Yaouanc, Jean-Jacques
Trégana
F-29263 Locmaria-Plouzane(FR)
Inventeur: Filali Zegzouti, Ayoub
25, rue de Saint-Brieuc
F-29200 Brest(FR)
Inventeur: Malouala, Denis
6, Impasse des Fauvettes
F-29490 Guipava(FR)
Inventeur: Des Abbayes, Hervé
3, rue de l'Eglise
F-29200 Brest(FR)
Inventeur: Clement, Jean-Claude
Castel-Huel
F-29870 Coat-Meal(FR)
Inventeur: Bernard, Hélène
Kérilliès
F-29880 Plouguerneau(FR)
Inventeur: Le Gall, Guénaelle
56 Hent Ménez-Land
F-29170 Fouesnant(FR)

(74) Mandataire: Ahner, Francis et al
CABINET REGIMBEAU, 26, avenue Kléber
F-75116 Paris(FR)

(54) **Procédé de préparation de tétramines cycliques monofonctionnalisées.**

(57) Procédé de préparation de tétramines cycliques monofonctionnalisées de formule I

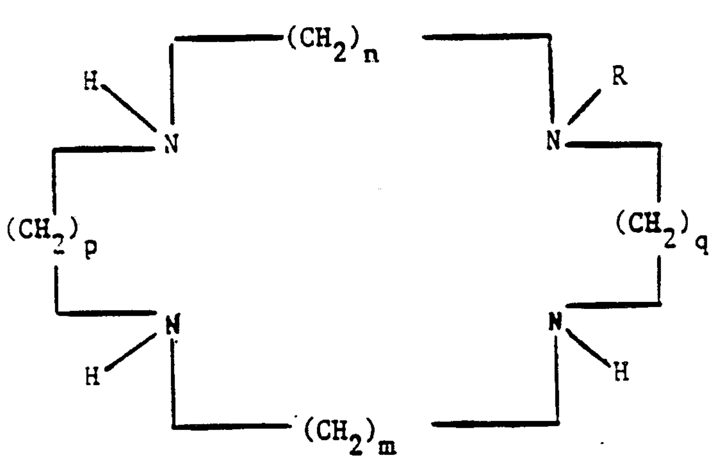

(I)

EP 0 389 359 A1

dans laquelle R représente un radical organique saturé ou non, notamment polymérisable, caractérisé en ce que l'on prépare le composé tétraazacycloalcane correspondant (selon la formule II), dans lequel trois des quatre atomes d'azote sont engagés par des liaisons covalentes avec un seul atome ou groupe d'atomes intracycliques A, en ce que l'on fait réagir ce composé triprotégé avec un composé organique RX (de formule III), X désignant un groupe nucléofuge, et en ce que l'on déprotège le composé tétraazacycloalcane triprotégé et monofonctionnalisé sur l'azote non protégé (selon la formule IV) ainsi obtenu.

## PROCEDE DE PREPARATION DE TETRAMINES CYCLIQUES MONOFONCTIONNALISEES

La présente invention, faite au Laboratoire de chimie, électrochimie et photochimie moléculaires de l'Université de Bretagne Occidentale, Laboratoire associé au Centre National de la Recherche Scientifique, concerne un procédé pour la préparation de tétramines cycliques monofonctionnalisées.

Les tétraazamacrocycles sont des coordinats macrocycliques qui présentent la particularité de former des complexes extrêmement stables avec les ions des métaux de transition, (de la colonne du manganèse à celle du zinc, mais aussi avec le plomb, etc). En revanche, ces molécules ne s'associent pas ou très peu aux alcalins et alcalino-terreux (Voir Host Guest Complex Chemistry I,II,III. F. VOGTLE et E. WEBER Edit., SPRINGER Verlag, 1980, 1981 et 1984 et Coordination Chemistry of Macrocyclic Compounds, A. MELSON Edit., PLENUM, 1979).

De nombreuses applications sont décrites dans la littérature dans des domaines aussi divers que l'électrochimie (électrodes modifiées), la catalyse, la stabilisation de degrés d'oxydation instables ou encore, la médecine nucléaire. Chacune de ces applications nécessite une modification spécifique du ligand tétraazoté et donc une reprise de sa synthèse.

De même, la très forte affinité des tétramines cycliques pour les métaux qui peuvent être des métaux rares ou précieux (Cu, Ag, Au, Pd, Ru, Rh, Os, etc) ou au contraire polluants (Zn, Cd, Hg, Pb, etc) est également une source potentielle d'applications intéressantes. Cependant, dans ce cas aussi, pour bénéficier des propriétés de ces molécules il est nécessaire de réaliser, pour chacune des applications envisagées, une modification spécifique de leur structure. Ainsi, si l'on désire utiliser une tétramine cyclique pour extraire un ion d'une phase aqueuse vers une phase organique, il est nécessaire de lui adjoindre un groupement lipophile qui accroîtra la solubilité du complexe dans les solvants organiques. De même, pour faire un résine échangeuse d'ion chélante, il faut fixer le macrocycle sur un polymère.

EP-A-0 287 436 illustre l'état de la technique considéré comme le plus proche. Le procédé de monofonctionnalisation décrit dans ce brevet antérieur a permis la préparation de dérivés connus du cyclam ainsi que d'autres nouveaux dérivés tant du cyclam que d'autres tétraazacycloalcane symétriques ou non.

La présente invention a précisément pour but la mise au point d'un nouveau mode de monofonctionnalisation de ce type de tétraazamacrocycles, qui puisse être mis en oeuvre dans des conditions moins contraignantes, plus économiques, et qui puisse donc être mieux exploité à l'échelon industriel.

Le nouveau procédé objet de la présente invention est fondé sur le blocage de trois des quatre atomes d'azote du cycle par engagement dans des liaisons covalentes avec un seul atome ou groupe d'atomes intracyclique.

Plus précisément, l'invention vise un procédé pour la préparation de tétramines monofonctionnalisées de formule I

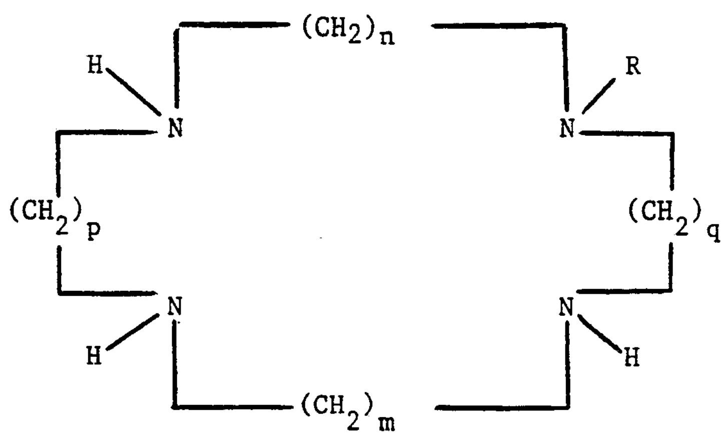

(I)

dans laquelle :
m = n = p = q = 2, ou
m = n = 2 ; p = q = 3, ou
m = 2 ; n = p = q = 3, ou

n = 2 ; m = p = q = 3, ou
m = n = p = q = 3, ou
m = n = 3 ; p = q = 4, et
R représente un radical organique saturé ou non, notamment polymérisable, caractérisé en ce que l'on prépare un composé tétraazacycloalcane triprotégé de formule II

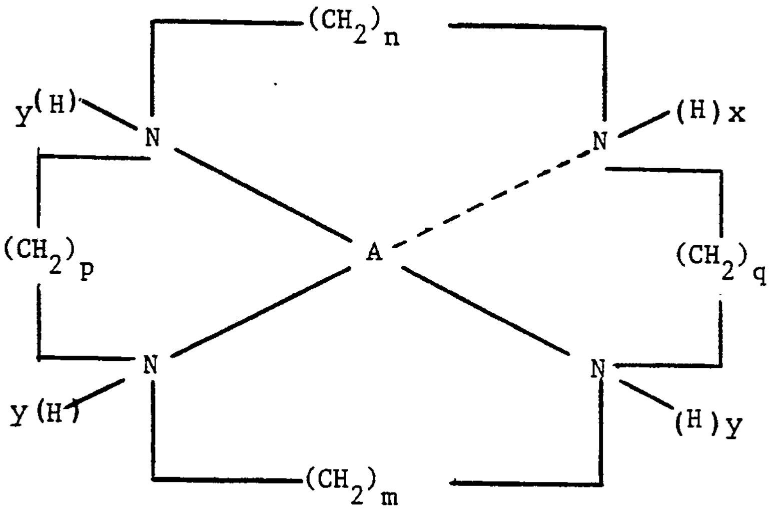

(II)

dans laquelle :
- x et y représentent indépendamment l'un de l'autre 1 ou 0,
- au moins trois des quatre atomes d'azote sont engagés, par des liaisons covalentes, avec un seul atome ou groupe d'atomes intracyclique A, et
- la ligne en pointillés représente une liaison covalente établie éventuellement entre le quatrième atome d'azote et A;

que l'on fait réagir le composé tétraazacycloalcane triprotégé de formule II avec un composé organique de formule III

R - X (III)

dans laquelle :
R a la signification donnée à propos de la formule I, et X désigne un groupe nucléofuge, notamment un halogène ou un radical tosylate, pour obtenir un composé tétraazacycloalcane triprotégé et monofonctionnalisé de formule IV

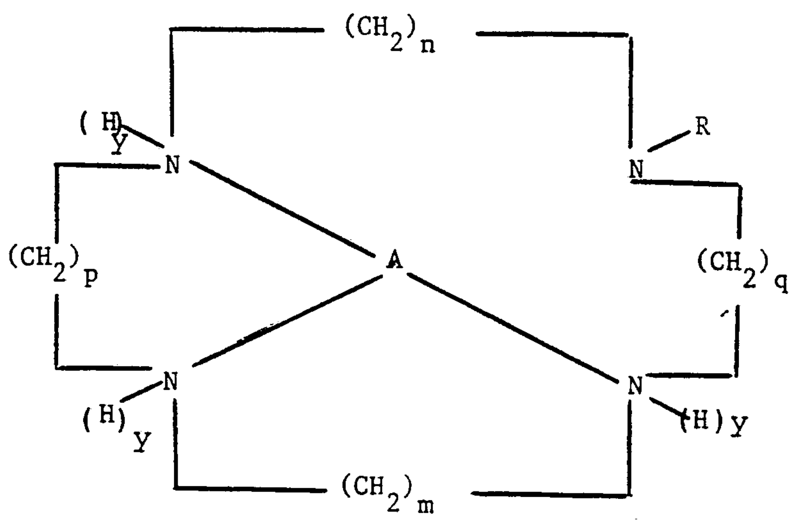

(IV)

et en ce que l'on déprotège le composé de formule IV pour obtenir le composé correspondant de formule I.

Dans les formules II et IV précédentes, le symbole A peut en particulier désigner un atome de bore, ou un groupe métal-carbonyle $M(CO)_3$ avec M choisi parmi Cr, Mo et W, ou encore un groupement P(O) ou P(+). Dans le cas où A désigne un groupement P(+), la liaison en pointillés de la formule II représente alors une liaison covalente établie entre P(+) et le quatrième atome d'azote.

On illustrera ci-après la première variante du procédé selon l'invention à l'aide d'un schéma réactionnel relatif à la monoalkylation du cyclam.

## VARIANTE A

La méthode utilisée pour réaliser de façon univoque la monoalkylation des tétraazamacrocycles met à profit la propriété des métaux carbonyles du 6ème groupe de former par réaction avec les amines, des complexes azotés par substitution d'un, puis deux, puis trois ligands C≡O.

Ce procédé de monoalkylation selon l'invention est très sélectif. Seul le composé de monoalkylation est obtenu. Ce procédé ne nécessite donc aucune étape supplémentaire de purification. Par ailleurs, les étapes de protection et de déprotection sont quantitatives et stoechiométriques.

## SCHEMA REACTIONNEL

a) Protection

La réaction d'un tétraazamacrocycle avec un métal carbonyle du 6ème groupe permet le blocage de trois des quatre atomes d'azote :

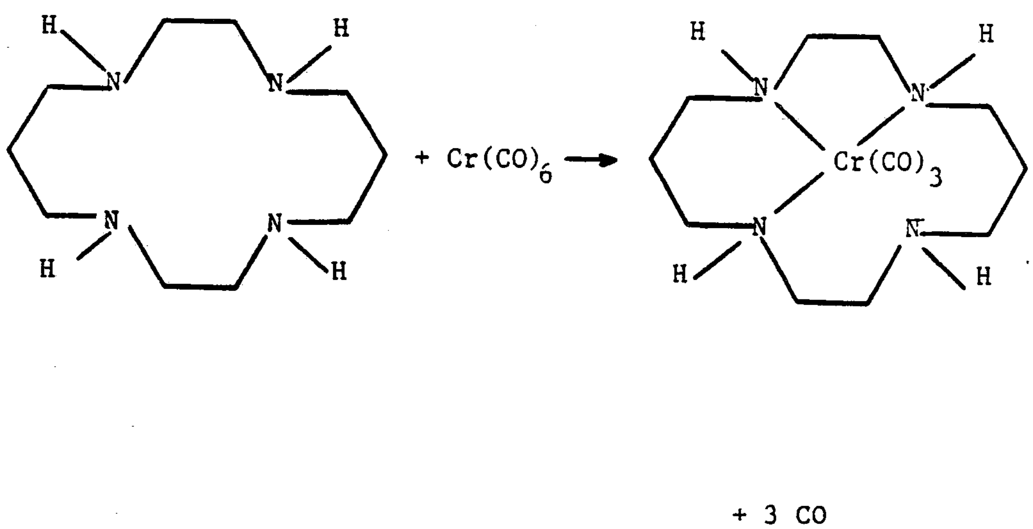


b) Alkylation

La réaction d'alkylation par les halogénures d'alkyle sur le 4ème atome d'azote demeuré libre s'effectue dans les conditions classiques de la $SN_2$ avec de bons rendements.

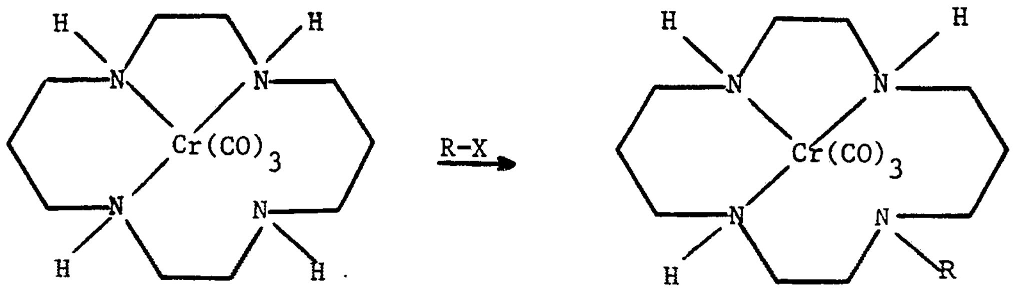


c) Déprotection

L'élimination du groupement protecteur $Cr(CO)_3$, est simplement réalisée par oxydation à l'air, à pH acide.

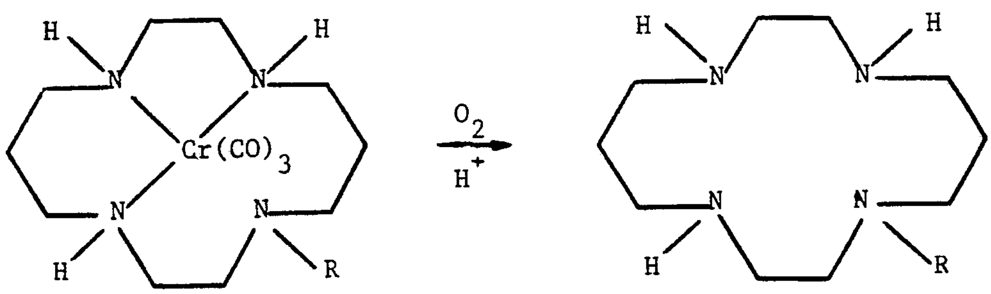


La seconde variante du procédé selon l'invention est également illustrée ci-après à l'aide d'un schéma réactionnel relatif à la monoalkylation du cyclam.

**VARIANTE B**

Cette variante est également fondée sur le blocage intracyclique de trois des quatre atomes d'azote du cyclam. L'intermédiaire triprotégé de formule II est le phosphorotriamide qui peut être obtenu par exemple selon le schéma ci-après, comme décrit par J.E. RICHMAN et J.J. KUBALE J. Am. Chem. Soc. 1983, 105, 749 :

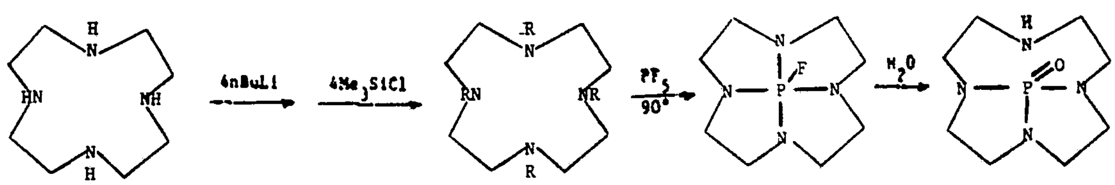


R = $SiMe_3$

De même, les phosphorotriamides intermédiaires de formule II peuvent être préparés dans d'excellentes conditions pratiques, en faisant réagir le cyclam sur $POCl_3$ en présence d'une base.

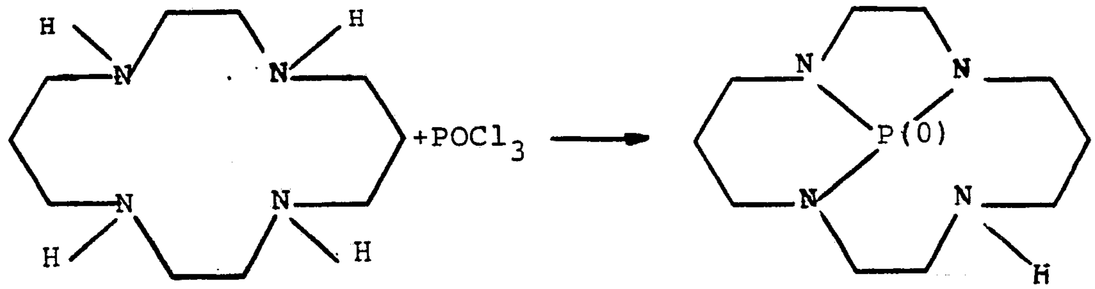


Toutefois, conformément à la présente invention, ces intermédiaires sont obtenus avec d'excellents rendements selon le schéma réactionnel ci-dessous, qui constitue une amélioration très notable du mode opératoire décrit précédemment.

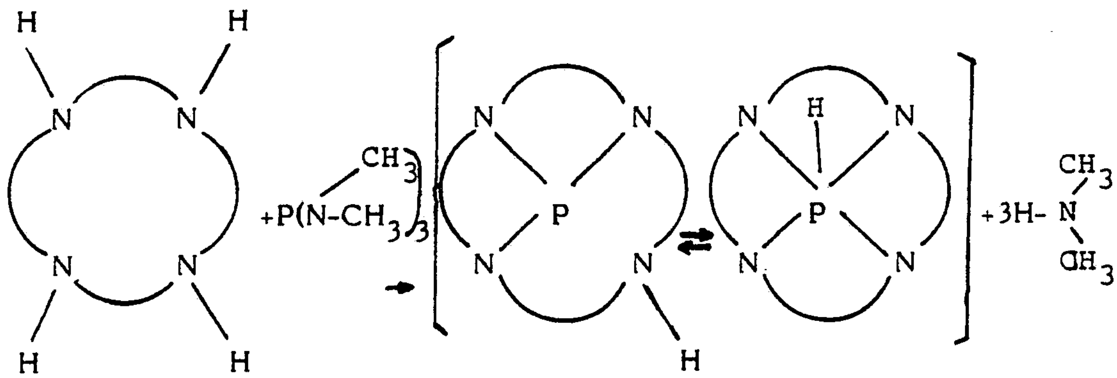

Intermédiaire 1

L'intermédiaire 1 a été étudié par J.E. RICHMAN et T.J. ATKINS (Tet. Lett. 1978,52, 5149) et ses caractéristiques sont connues, en particulier, il s'agit de deux formes en équilibre plus ou moins déplacé. Cet intermédiaire 1 est obtenu facilement par simple chauffage dans un solvant organique (par exemple le toluène); si on opère sous courant d'azote sec, la diméthylamine formée est entraînée et la réaction est complète au bout de quelques heures. L'avancement de la réaction peut être suivi par dégagement de l'amine formée dans une quantité stoechiométrique d'acide contenant un peu d'indicateur coloré. Au virage de l'indicateur, la réaction est terminée.

L'intermédiaire 1 est traité par $CCl_4$ en excès. Il se forme alors l'intermédiaire 2 de manière quantitative:

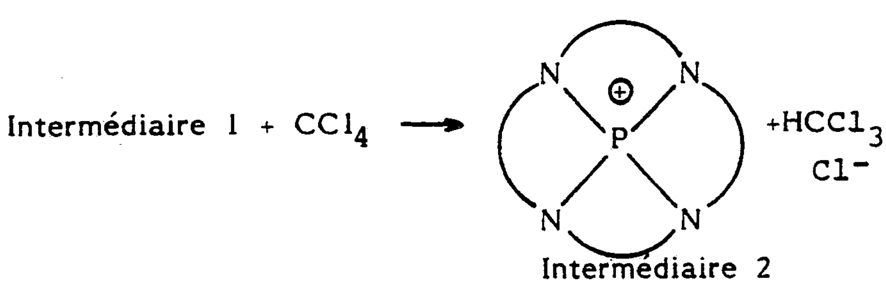


Intermédiaire 2

L'hydrolyse de cet intermédiaire 2 est réalisée par la soude diluée :

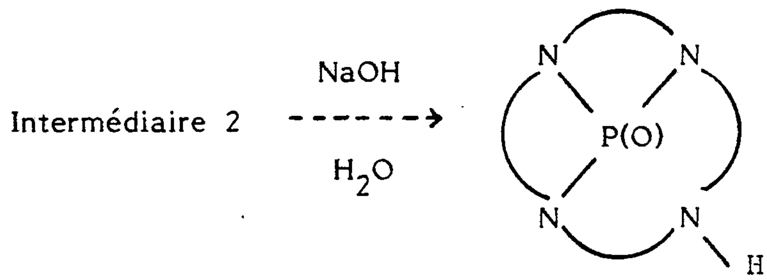


L'ensemble de ces opérations est quantitatif; le macrocycle triprotégé par un P(O) est extrait par un solvant organique (dichlorométhane) pour être ensuite utilisé tel que dans l'étape de monoalkylation.

Le phosphorotriamide intermédiaire s'alkyle très facilement dans les conditions classiques de la réaction $SN_2$. La libération de la tétramine de son groupement protecteur se fait facilement en milieu acide, au reflux pendant quelques heures. C'est ainsi que la monofonctionnalisation est réalisée de préférence dans le DMF, en présence d'une base, de préférence le carbonate de sodium ou de potassium. Quant à la déprotection,elle, est réalisée avec HCl 3M.

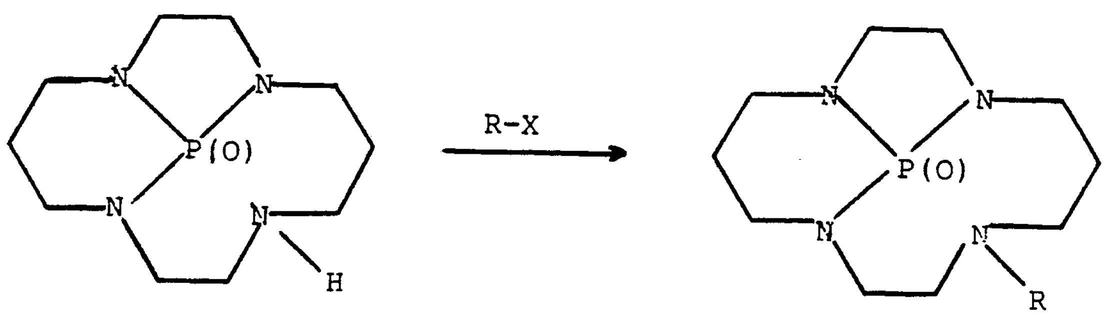


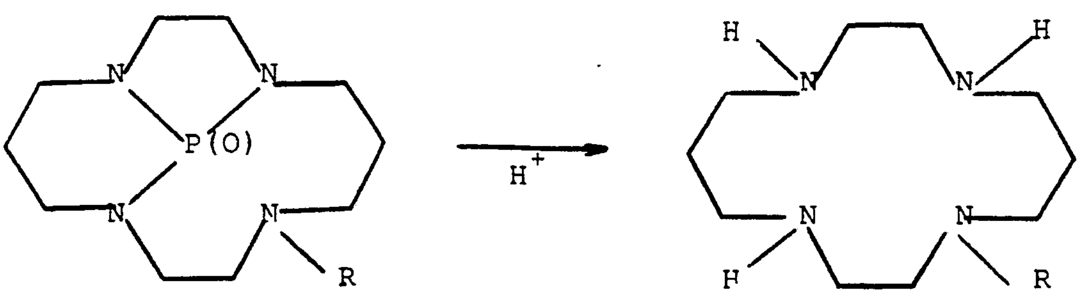


Par ailleurs, lorsqu'une fonction alcool est portée par un carbone lié à un autre carbone insaturé, cet alcool activé, par exemple l'alcool benzylique, peut directement réagir avec l'intermédiaire 2.

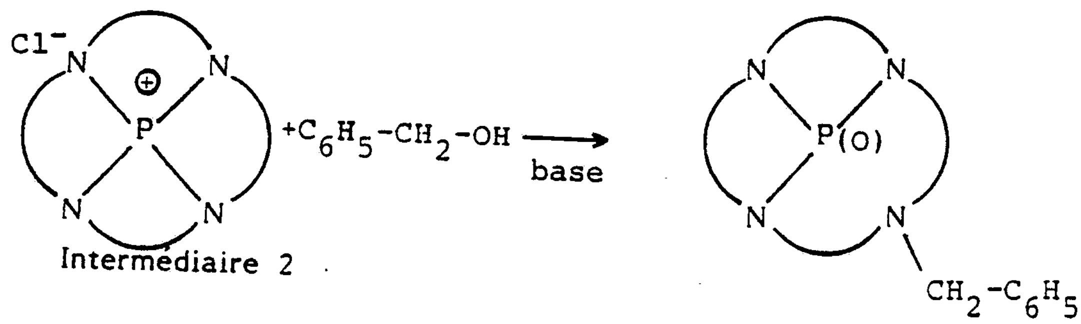


Intermédiaire 2

Le rendement de cette étape est également quantitatif et constitue une alternative intéressante de la variante B.

## VARIANTE C

La troisième variante du procédé selon l'invention consiste à préparer un intermédiaire de formule générale II triprotégé par un atome de bore intracyclique.

Cet intermédiaire triprotégé peut être obtenu de différentes manières. On peut par exemple l'obtenir par une réaction de transamination à l'aide de tris-diméthylamino-borane.

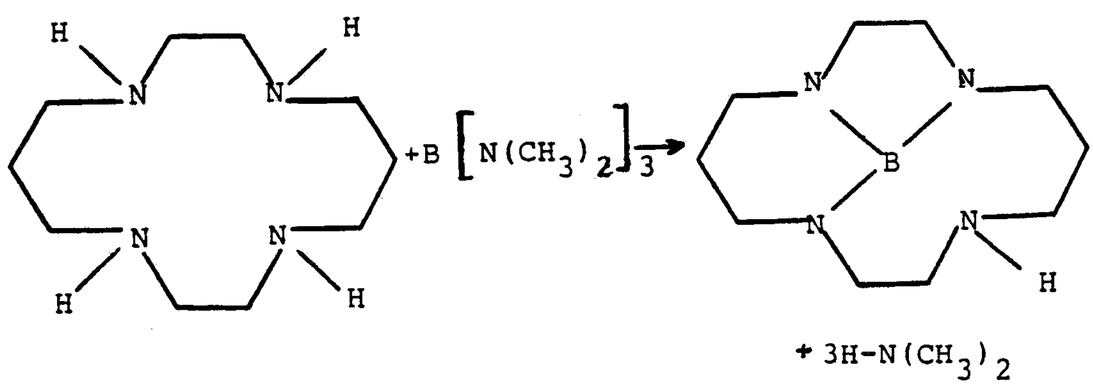


La réaction est menée dans un solvant organique, par exemple le toluène, le dégagement de l'amine peut, là encore, permettre de suivre la réaction.

Dans le mode opératoire général, le solvant est évaporé, on reprend le résidu par un solvant polaire par exemple, le THF et on ajoute une quantité équivalente de n-butyl-lithium. L'anion ainsi formé s'alkyle dans d'excellentes conditions.

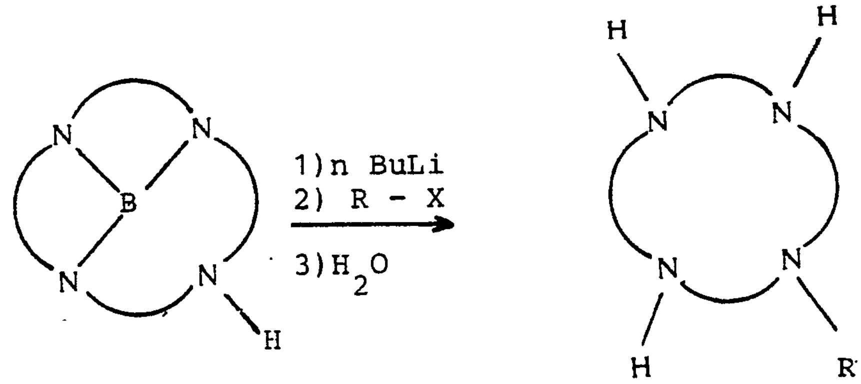


Le principal intérêt de cette variante réside dans l'étape de déprotection qui se fait très rapidement dans des conditions très douces: $H_2O$ suffit, néanmoins, on se place en milieu basique pour faciliter l'extraction du tétraazamacrocyle.

On notera que le tris-diméthylamino-borane peut également être remplacé par l'orthoborate de méthyle $B(OCH_3)_3$ ou d'isopropyle.

En résumé, chacune de ces méthodes possède son domaine d'application propre, néanmoins, nous soulignons que dans tous les cas, il s'agit de méthodes stoechiométriques ne nécessitant pas un excès de ligand. Les exemples présentés ci-après montrent que la triprotection est très efficace, l'azote restant libre peut être engagé d'une manière générale dans toutes les réactions habituelles d'alkylation des amines secondaires. La variante C est tout à fait originale et permet d'introduire des fonctions qui ne résisteraient pas à une hydrolyse acide prolongée. Ainsi le dérivé monomérique cyclam-$CH_2$-$C_6H_4$-$CH=CH_2$ est préparé par cette seule voie, avec des rendements supérieurs. Nous insistons également sur la rapidité de cette dernière méthode qui permet d'obtenir une mono N-alkylation complète dans un délai de quelques heures. Dans tous les cas, les produits sont de qualité satisfaisante et ne nécessitent pas d'étape de purification ultérieure.

Le procédé objet de la présente invention sera illustré ci-après à l'aide d'exemples de mise en oeuvre particuliers.

EXEMPLE 1

Toutes ces manipulations sont effectuées à l'abri de l'air, sous azote.

*** Préparation du complexe chrome tricarbonyle du cyclam**

Dans 50ml de dibutyléther sec et désaéré, on place 1g de cyclam (5 mmoles) et 1,38g de chrome hexacarbonyle (6 mmoles) ; le mélange est chauffé à la température de reflux du solvant pendant 2-3 heures. Le complexe cyclam $Cr(CO)_3$ précipite. Après refroidissement à température ambiante, le solvant est éliminé par filtration sous azote et le solide est rincé à l'éther diéthylique puis séché sous vide à 40° C; l'excès de $Cr(CO)_6$ est ainsi éliminé.

*** Alkylation et déprotection**

Dans 5ml de DMF sec et désaéré, on place 0,278g de cyclam $Cr(CO)_3$ (0,8 mmole), 96µl de bromure de benzyle (0,8 mmoles) et un excès de carbonate de sodium. Le mélange est chauffé à 100° C pendant 2 heures puis refroidi à température ambiante ; le solvant est évaporé sous vide et ce résidu est repris dans 20ml de HCl 6N puis oxydé à l'air. Le mélange devient vert. Le mélange est basifié à pH = 14 par addition de potasse en pastilles puis extrait par 2 x 25 ml de $CH_2Cl_2$ ; après séchage des phases organiques le solvant est évaporé ; le résidu huileux est repris dans 5ml d'éthanol sulfurique à 10% ; le précipité est filtré, repris dans 5ml d'eau, basifié puis à nouveau extrait pour donner 0,176g de N-benzylcyclam (62%) pur.

Des résultats entièrement identiques ont été obtenus en remplaçant le chrome hexacarbonyle par du molybdène hexacarbonyle ou du tungstène hexacarbonyle.

EXEMPLE 2

*** Préparation du complexe**

Dans 40 ml de toluène, on place 200 mg de cyclam (1 mmole) et on ajoute 1 mmole de tris-(diméthylamino)-phosphine. Le mélange est porté à 100° C. Cette température est maintenue jusqu'à dégagement de 3 mmoles de diméthylamine. Après refroidissement, on ajoute lentement 1 ml de $CCl_4$ sec. L'intermédiaire précipite. L'hydrolyse par la soude 4M suivi d'une extraction au dichlorométhane permet de récupérer quantitativement le complexe cyclam P(O)(R 98%).

*** Alkylation et déprotection**

Dans 30 ml de DMF sec contenant le composé précédent, on ajoute 300mg de carbonate de sodium. Le mélange est porté à 100° C, et on ajoute 130 µl (1 mmole) de bromure de benzyle. Après une heure de réaction, le mélange est filtré, le solvant évaporé, puis le résidu est repris par 15 ml d'acide chlorhydrique 3M et chauffé pendant dix heures. Après refroidissement, la solution est rendue basique par addition de soude, et on extrait plusieurs fois la solution aqueuse par le dichlorométhane. Les phases organiques sont séchées et le solvant chassé par distillation. On obtient ainsi le N-benzyl cyclam avec un rendement de 95%.

EXEMPLE 3

Les manipulations sont effectuées à l'abri de l'air, sous azote, jusqu'à l'étape de déprotection.

*** Préparation du complexe**

Dans 40 ml de toluène, on place 200 mg de cyclam (1 mmole), on ajoute 190 µl de tris-(diméthylamino)borane. Le mélange est porté à 100° C. Cette température est maintenue jusqu'à dégagement de 3 mmoles de diméthylamine.

*** Alkylation et déprotection**

Le toluène est évaporé et remplacé par 30 ml de THF sec. Après dissolution du complexe, le mélange est refroidi à -30° C, puis on additionne une quantité équivalente de n-butyl-lithium. Le mélange est maintenu sous agitation à -30° C pendant 15 mn. Après addition de 130 µl de bromure de benzyle (1 mmole), on laisse revenir à température ambiante; l'agitation est maintenue pendant environ 2 heures.

Le complexe est détruit en additionnant quelques gouttes d'eau.

Le THF est éliminé par distillation sous vide. Le résidu est traité par la soude à 30%, puis extrait par 2 fois 25 ml de $CH_2Cl_2$. Après séchage des phases organiques, le solvant est évaporé. Le composé de mono-alkylation se présente sous forme d'une huile jaune. Le rendement = 95%.

Chacune des variantes décrites en détail dans le cadre des exemples 1 à 3 précités a pu être utilisée de façon satisfaisante pour obtenir les composés suivants. L'halogénure utilisé peut être choisi parmi le chlorure, un bromure, un iodure ou encore un tosylate d'alkyle, d'aryle ou d'aralkyle.

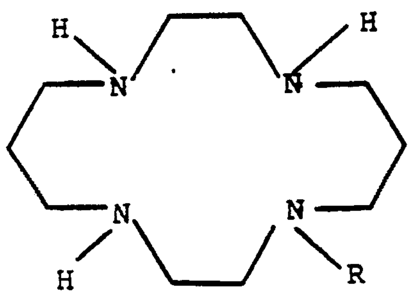

et

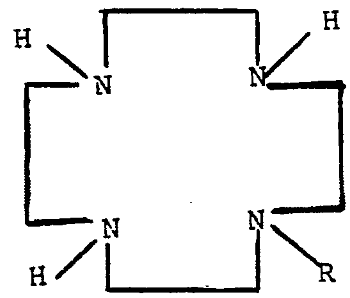

Dans lesquels :

R = -$CH_2$-Ø

R = -$CH_2$-$CH=CH_2$

R = $nC_{12}H_{25}$

R = -$CH_3$

R = -$(CH_2)_3$-Ø

R = $CH_2$-Ferrocène

R = $CH_2$-$C_6H_4$-$CH=CH_2$ (isomère méta + para).

Le composé ci-après a été obtenu en mettant en présence le complexe et le dihalogénure organique correspondant dans les proportions 2:1 ainsi que ses deux autres isomères.

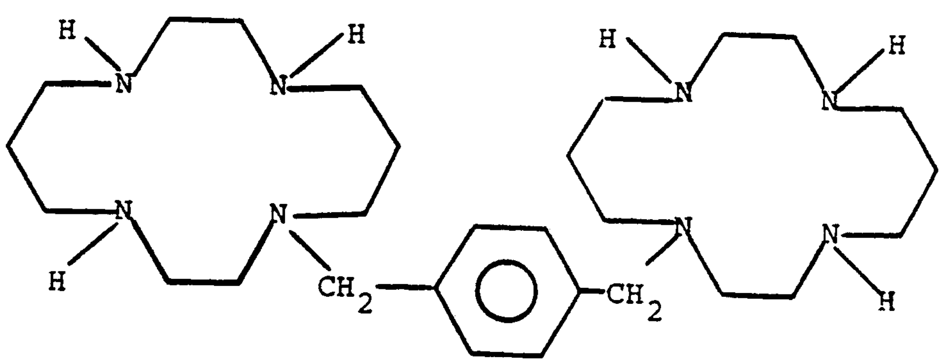

Le tableau I présenté ci-après rassemble les résultats obtenus.

TABLEAU I

| COMPOSE | RENDEMENT | VARIANTE | AGENT ALKYLANT |
|---|---|---|---|
| Dérivés du (2222) | 95% | A:Cr(CO)$_3$L | $C_6H_5CH_2Br$ |
| 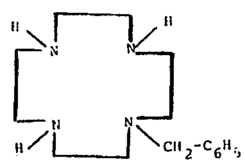 | 75% | A:Mo(CO)$_3$L | " |
| | 70% | A:W(CO)$_3$L | " |
| | 90% | B | " |
| | 50% | C | " |
| Dérivés du 2323 | | | |
| 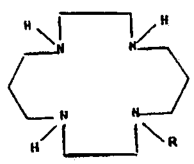 | | | |
| $R=CH_2-C_6H_5$ | 97% | A:Cr(CO)$_3$L | $C_6H_5CH_2Br$ |
| | 87% | A:Mo(CO)$_3$L | " |
| | 85% | A:W(CO)$_3$L | " |
| | 80% | B | $C_6H_5CH_2OH$ |
| | 95% | B | $C_6H_5CH_2Br$ |
| | 95% | C | " |
| $R = CH_3$ | 30% | A:Cr(CO)$_3$L | $CH_3I$ |
| | 30% | B | $CH_3I$ |
| | 80% | C | $CH_3$-O-Ts |
| $R=-(CH_2)_3-C_6H_5$ | 75% | B | R-Br |
| | 90% | C | R-OTs |

TABLEAU I (Suite 1)

| COMPOSE | RENDEMENT | VARIANTE | AGENT ALKYLANT |
|---|---|---|---|
| R=$-C_{12}H_{25}$ | 30% | A:$Cr(CO)_3L$ | R-Br |
| | 70% | B | R-Br |
| R=$-CH_2-CH=CH_2$ | 55% | A:$Cr(CO)_3L$ | R-Br |
| | 70% | B | R-OH |
| | 80% | B | R-OH |
| | 80% | C | R-Br |
| R=$-CH_2$-Ferrocène | 80% | A:$Cr(CO)_3L$ | R-Cl |
| | 80% | B | R-OH |
| | 80% | B | R-Cl |
| | 80% | C | R-Cl |
| R=$-CH_2-C_6H_4-CH=CH_2$ Mélange commercial d'isomères méta+para | 95% | C | R-Cl |
| Dérivés bicycliques 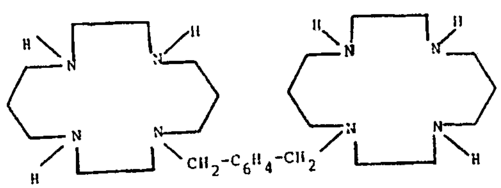 | | | |

TABLEAU I (Suite 2)

| COMPOSE | | RENDEMENT | VARIANTE | AGENT ALKYLANT |
|---|---|---|---|---|
| Isomère | ortho | 45% | B | $BrCH_2-C_6H_4-CH_2Br$ |
| | méta | 80% | B | " |
| | para | 90% | $A:Cr(CO)_3L$ | " |
| | para | 90% | B | " |
| Dérivé du 3333 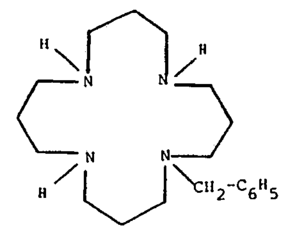 | | 90% | B | $C_6H_5CH_2Br$ |
| | | 80% | C | " |
| Dérivé du 3434 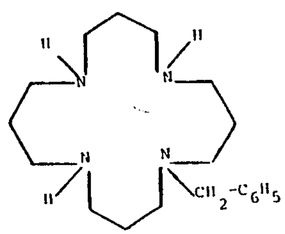 | | 90% | B | $C_6H_5CH_2Br$ |
| | | 70% | C | " |

## Revendications

1°) Procédé de préparation de tétramines cycliques monofonctionnalisées de formule I

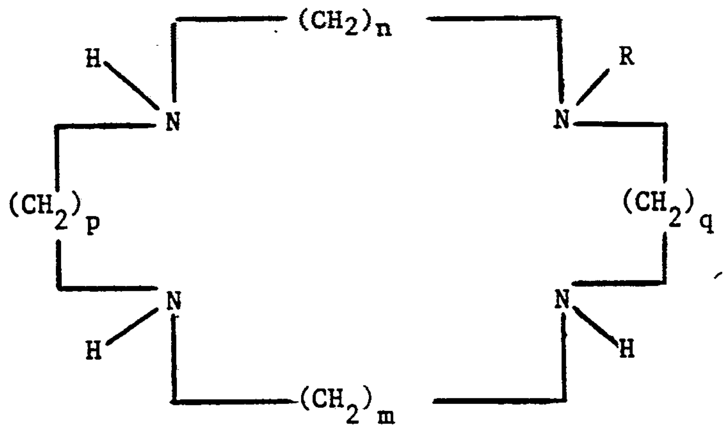

(I)

dans laquelle :
m = n = p = q = 2, ou
m = n = 2 ; p = q = 3, ou
m = 2 ; n = p = q = 3, ou
n = 2 ; m = p = q = 3, ou
m = n = p = q = 3, ou
m = n = 3 ; p = q = 4, et
R représente un radical organique saturé ou non, notamment polymérisable, caractérisé en ce que l'on prépare un composé tétraazacycloalcane triprotégé de formule II

(II)

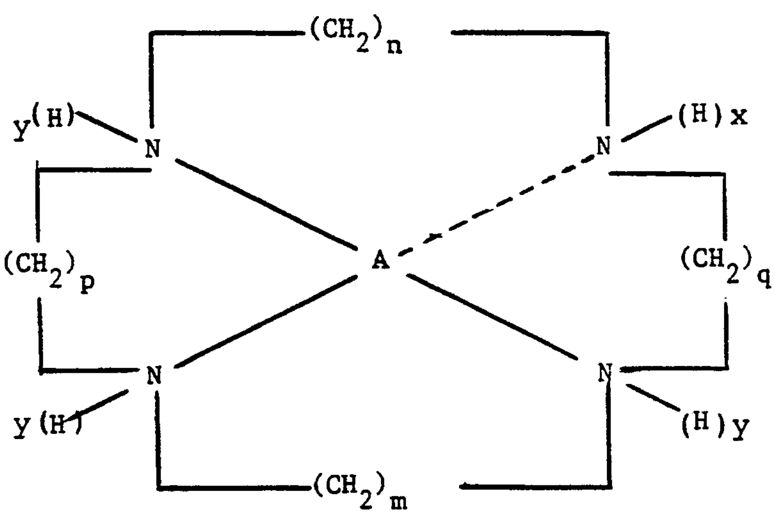

dans laquelle :
- x et y représentent indépendamment l'un de l'autre 1 ou 0,
- au moins trois des quatre atomes d'azote sont engagés, par des liaisons covalentes, avec un seul atome ou groupe d'atomes intracyclique A, et
- la ligne en pointillés représente une liaison covalente établie éventuellement entre le quatrième atome

d'azote et A, que l'on fait réagir le composé tétraazacycloalcane triprotégé de formule II avec un composé organique de formule III

R - X (III)

dans laquelle :

R a la signification donnée à propos de la formule I, et X désigne un groupe nucléofuge, notamment un halogène ou un radical tosylate, pour obtenir un composé tétraazacycloalcane triprotégé et monofonctionnalisé de formule IV

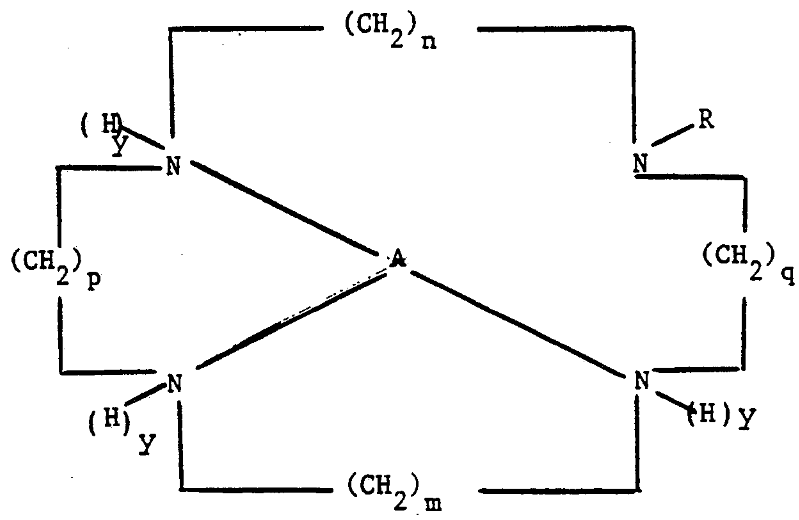

(IV)

et en ce que l'on déprotège le composé de formule IV pour obtenir le composé correspondant de formule I.

2°) Procédé selon la revendication 1, caractérisé en ce que dans les formules II et IV, le symbole A désigne

- un atome de bore,
- un groupe métal-carbonyle $M(CO)_3$ où M est choisi parmi Cr, Mo et W, ou
- un groupement P(O) ou P(+).

3°) Procédé selon l'une des revendications 1 et 2, caractérisé en ce que l'on prépare un composé de formule II dans laquelle A représente $Cr(CO)_3$ et, après monofonctionnalisation, on déprotège le complexe chrometricarbonyle par oxydation à l'air et à pH acide.

4°) Procédé selon la revendication 3, caractérisé en ce que le composé de formule II dans laquelle A représente $Cr(CO)_3$ est obtenu par réaction du composé tétraazacycloalcane correspondant de formule I avec du chrome hexacarbonyle.

5°) Procédé selon l'une des revendications 1 et 2, caractérisé en ce que l'on prépare un composé de formule II dans laquelle A représente P(O) et, après monofonctionnalisation, on déprotège le composé phosphorotriamide en milieu acide.

6°) Procédé selon la revendication 5 caractérisé en ce que le composé de formule II dans laquelle A représente P(O) est obtenu par réaction du composé tétraazacycloalcane correspondant de formule I avec $P(N(CH_3)_2)_3$, traitement au $CCl_4$ dudit intermédiaire formé puis hydrolyse basique.

7°) Procédé selon l'une des revendications 1 et 2 caractérisé en ce que l'on prépare un composé de formule II dans laquelle A représente un groupement P(+) lié à chacun des 4 atomes d'azote par une liaison covalente et après monofonctionnalisation, on déprotège le composé phosphorotriamide en milieu acide.

8°) Procédé selon la revendication 7 caractérisé en ce que le composé de formule II dans laquelle A représente P(+) lié par une liaison covalente à chacun des quatre atomes d'azote est obtenu par réaction du composé tétraazacycloalcane correspondant de formule I, avec $P(N(CH_3)_2)_3$ puis traitement au $CCl_4$ dudit intermédiaire obtenu.

9°) Procédé selon l'une des revendications 1 et 2, caractérisé en ce que l'on prépare un composé de formule II dans laquelle A représente un atome de bore et, après monofonctionnalisation, on déprotège le composé borotriamine par simple hydrolyse.

10°) Procédé selon la revendication 9, caractérisé en ce que le composé de formule II dans laquelle A représente un atome de bore est obtenu par réaction du composé tétraazaycloalcane correspondant de

formule I avec du tris-diméthylamino-borane $B[N(CH_3)_2]_3$ ou de l'orthoborate de méthyle $B(OCH_3)_3$.

11°) Procédé selon l'une des revendications 1 à 10, caractérisé en ce que le composé organique de formule III est un dihalogénure organique conduisant à la formation d'un composé de formule I bimacrocyclique.

12°) Procédé selon la revendication 11, caractérisé en ce que le composé organique de formule III est le 1,4 le 1,2 ou le 1,3-di(bromométhyl)-benzène.

13°) A titre d'intermédiaires de synthèse, les composés tétraazacycloalcane triprotégés de formule II selon l'une des revendications 1 à 10.

Office européen des brevets

# RAPPORT DE RECHERCHE EUROPEENNE

Numero de la demande

EP 90 40 0762

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | CHEMICAL ABSTRACTS, vol. 96, no. 11, 15 mars 1982, page 600, résumé no. 85658a, Columbus, Ohio, US; J.E. RICHMAN et al.: "NMR characterization of homologous cyclic phosphoramides", & ACS SYMP. SER. 1981, 171(Phosphorus Chem.), 271-4<br>* Extrait *<br>--- | 13 | C 07 D 257/02<br>C 07 F 9/652<br>C 07 F 11/00 |
| Y | SYNTH. REACT. INORG. MET.-ORG. CHEM., vol. 17, no. 3, 1987, pages 301-306, Marcel Dekker, Inc.; F. BOUVIER et al.: "Synthesis and characterisation of cyclamphosphane sulfide and selenide"<br>* En entier *<br>--- | 1,13 | |
| D,A | TETRAHEDRON LETTERS, no. 52, 1978, pages 5149-5152, Pergamon Press, Ltd, GB; T.J. ATKINS et al.: "Polyaminophosphoranes III. P(III) - P(V) tautomerism in four nitrogen cage phosphoranes"<br>* En entier *<br>--- | 1,13 | **DOMAINES TECHNIQUES RECHERCHES (Int. Cl.5)** |
| A | JOURNAL OF THE AMERICAN CHEMICAL SOCIETY, vol. 108, no. 6, 19 mars 1986, pages 1167-1173, American Chemical Society, US; J.-M. DUPART et al.: "Coordination chemistry of cyclamphosphorane. Access to transition-metal cyclamphosphoranides. Crystal and molecular structure of CpMo(CO)2(cyclamphosphoranide)"<br>* En entier *<br>--- -/- | 1,13 | C 07 D 257/00<br>C 07 F 9/00<br>C 07 F 11/00 |

Le présent rapport a été établi pour toutes les revendications

| Lieu de la recherche | Date d'achèvement de la recherche | Examinateur |
|---|---|---|
| LA HAYE | 21-05-1990 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numero de la demande

EP 90 40 0762

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 107, no. 8, 24 août 1987, page 714, résumé no. 69559j, Columbus, Ohio, US; F. BOUVIER et al.: "Improved synthesis of cyclamphosphine oxide, a potential bidentate asymmetric ligand. Synthesis and characterization of some of its rhodium complexes. Molecular and crystal structure of (cyclamphosphine oxide)dicarbonylchlororhodium (I)", & INORG. CHEM. 1987, 26(13), 2090-3 * Extrait * | 1,13 | |
| A | FR-A-2 613 718 (CNRS) * Revendications * & EP-A-0 287 436 (Cat. D) | 1 | |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 21-05-1990 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)